# EUROPEAN PATENT SPECIFICATION

(11) **EP 2 187 985 B1**
(45) Date of publication and mention of the grant of the patent: **28.09.2016**
(21) Application number: 08788312.0
(22) Date of filing: 14.08.2008
(51) Int. Cl.: A61L 27/36, A61L 27/38

(54) **SCAFFOLDS**
GERÜSTE
ÉCHAFAUDAGES

(30) Priority: 14.08.2007 GB 0715806; 07.11.2007 GB 0721840; 13.05.2008 GB 0808645
(43) Date of publication of application: 26.05.2010
(73) Proprietor: Smith & Nephew PLC, London WC2N 6LA (GB)
(72) Inventor: LANGFORD, Kelly, Heslington, York YO10 5DF (GB); BURDON, Drew, Heslington, York YO10 5DF (GB); WHYTE, Muna, Heslington, York YO10 5DF (GB)
(74) Representative: Smith & Nephew
(86) International application number: PCT/GB2008/002743
(87) International publication number: WO 2009/022133

(56) References cited:
- US-A1- 2004 203 146
- US-A1- 2006 128 012
- US-A1- 2006 153 815

## Description

### FIELD OF THE INVENTION

The present invention finds applicability in the field of tissue culture as well as in the field of preparing tissue substitutes for tissue replacement.

### BACKGROUND

Articular cartilage consists of highly specialised chondrocytes surrounded by a dense extracellular matrix consisting mainly of type II collagen, proteoglycan and water. This avascular tissue has a limited ability of repair. Damage of cartilage produced by disease, such as rheumatoid arthritis and/or arthritis, or trauma can lead to serious physical deformity and debilitation.

Previous tissue engineering solutions for cartilage repair have focused around the production of a continuous piece of cartilage capable of repairing a cartilage defect in its entirety. This approach has significant drawbacks.

The size of the piece of cartilage which can be produced *in vitro* is limited. Large scale cell-scaffold cultures have limitations in terms of controlling proliferation and differentiation. These problems are primarily caused by insufficient nutrient diffusion through the scaffold, causing cell necrosis and areas of undifferentiated cells within the scaffold.

A further problem is that the dimensions of cartilage defects vary from patient to patient. As a consequence, pieces of cartilage have to be tailored to an implant site, multiple pieces of cartilage have to be tessellated in order to repair a large defect or the technique referred to as 'mosaicplasty' is used which requires the defect to be prepared to enable a standard sized cartilage replacement implant to be inserted. Other examples of forming tissue are disclosed in US2006/0128012, US2006/0153815 and US2004/0203146.

The use of cell microcarriers for fabricating cell-containing implants has addressed some of these issues. Typically cells are seeded onto resorbable microcarriers, such as spherical beads, cubes, cylinders or plates at low density and then culture-expanded to form aggregates. These cell micro-carrier aggregates can then be formulated as an injectable dispersion of aggregates or as a solid structure of consolidated aggregates prepared by further culturing of aggregates in a mold device having a geometry reflecting that of the defect. However a significant drawback with this technique is the reliance on the adherence of the cells to the micro-carrier surface. It is often necessary to coat the surface with, for instance, a bioactive peptide in order to improve the adhesion. Additionally, because there is often miminal resorption of the microcarrier during the culture expansion step, with significant rates of resorption only occurring once the microcarrier is implanted, this can have a significant influence on the healing process.

### SUMMARY OF THE INVENTION

According to a first aspect of the invention there is provided a method of preparing a tissue implant *in vitro,* said method comprising the steps of;
(a) providing cells isolated from a suitable tissue source;
(b) seeding at least two primary scaffolds with the cells and culturing these scaffolds for a period of time sufficient for the cells to secrete an extracellular matrix and;
(c) loading the at least two scaffolds obtained in (b) into a secondary scaffold.

An example of a suitably sized scaffold has a diameter of about 1-3mm and a depth of about 0.5-3mm, more particularly a diameter of about 1-2mm and a depth of about 0.5-1 mm.

The primary scaffolds can be of any suitable shape, for a cylinder, a disc or a cube.

The cells are seeded, preferably uniformly, onto a primary scaffold with typically between about 50,000 and 1x10⁶ cells per scaffold, or between about 100,000 and 300,000 cells per scaffold or more particularly between about 150,000 and 250,000 cells per scaffold.

A typical seeding density on a scaffold is between about 100,000 and 3 x10⁶ cells/mm³, or between about 300,000 and 800,000 cells/mm³ or more particularly between about 400,000 and 700,000 cells/mm³.

In specific embodiments of the invention the typical seeding density of cells on a primary scaffold is about 650,000 cells/mm³.

The cells may be seeded onto scaffolds using methods known to those skilled in the art. These include, but are not limited to, pipetting a suspension of cells onto the scaffold, incubating the scaffold in a suspension of cells for long enough that a proportion of the cells adhere to at least part of the surface of the scaffold or by combining the scaffolds and cells in a liquid and centrifuging to facilitate contact between the two components.

These cell-seeded scaffolds are then cultured for a period sufficient for the cells to produce cell aggregates. The primary scaffolds can be cultured in, for example, a conical flask, a roller bottle, a techne flask or a bioreactor.

In embodiments of the invention the cells are cultured on the scaffold for at least 12 hours to ensure appropriate aggregate formation, with continuous flow in a low volume of media. This low volume of culture media is relative to the number of scaffolds being cultured in any particular size of culture vessel and encourages aggregate formation by bringing the cell seeded scaffolds into close contact with each other. The volume of media is then increased and the cell-seeded scaffolds cultured under static conditions or with intermittent or constant agitation. Following an appropriate incubation period the at least two cell-seeded primary scaffolds are loaded into a secondary scaffold. This secondary scaffold can then be directly implanted into the site within a subject or alternatively cultured prior to implantation.

The incubation of the secondary scaffold prior to implantation allows the primary scaffolds to fuse into a larger aggregate which may be more stable at the time of implantation. These scaffolds are typically incubated for between 12 hours and 7 days.

The use of a secondary scaffold to contain the primary scaffolds provides additional mechanical strength and protection for the primary scaffolds during implantation and additionally while the tissue is being regenerated or repaired.

The second scaffold can be designed in various configurations, including but not limited to, cups, discs, cubes and cylinders.

The secondary scaffold may be reinforced to provide extra mechanical strength, for example with glass or polymeric fibres.

A drawback associated with a number of the prior art scaffolds used as the basis for tissue implants, for example non-woven and woven felts, ceramics, sponges, is the addition of a level of manufacturing complexity and cost into the production process. The use of fibres is predicted to reduce the complexity of manufacturing and therefore the costs. The use of fibres also allows for the development of more high-throughput seeding techniques.

Therefore according to the disclosure there is provided a method of preparing a tissue implant *in vitro,* said method comprising the steps of;
(a) providing cells isolated from a suitable tissue source,
(b) providing a plurality of fibres, and;
(c) culturing the cells and fibres together for a period of time sufficient for the cells to secrete an extracellular matrix and form a cellular aggregate.

According to the disclosure there is provided a method of augmenting, repairing or regenerating tissue within a subject, said method comprising the steps of:
(a) obtaining cells from a suitable tissue source;
(b) providing a plurality of fibres,
(c) culturing the cells and fibres together for a period of time sufficient for the cells to secrete an extracellular matrix and form a cellular aggregate, and;
(d) implanting the aggregate into a site within the subject.

An example of a suitably sized fibre has a diameter of about 10-100µm and a length of about 0.5-5mm, more particularly a diameter of about 10-100µm and a length of about 0.5-3mm, even more preferably a diameter of about 10-100µm and a length of about 1-3mm.

If it is desired that the scaffolds are of a substantially cylindrical shape, then the diameter of the fibre is preferably half its length.

The cells can be mixed with the fibres using methods known to those skilled in the art. These include, but are not limited to, pipetting a suspension of cells onto the fibres, incubating the fibres in a suspension of cells for long enough that a proportion of the cells adhere to at least part of the surface of the fibres or by combining the fibres and cells in a liquid and centrifuging to facilitate contact between the two components.

This cell/fibre mixture is then cultured for a period sufficient for the cells to secrete an extracellular matrix and form cellular aggregates. Culturing can take place in, for example, a conical flask, a roller bottle, a techne flask or a bioreactor.

In embodiments of the disclosure the cells are cultured with the fibres for at least 12 hours to ensure appropriate aggregate formation, with continuous-flow in a low volume of media. This low volume of culture media is relative to the number of fibres being cultured in any particular size of culture vessel and encourages aggregate formation by bringing the cells and fibres into close contact with each other. The volume of media is then increased and the cell-fibre aggregates are cultured under static conditions or with intermittent or constant agitation.

It is preferable that the fibrous material has completely resorbed by the end of the culture period or that only a residual amount of fibrous material remains, with the fibrous material having been replaced by secreted extracellular matrix.

The fibres function as a scaffold for the cells during the aggregation of the cells.

A typical cell seeding density is from about 2 x10⁴ to about 3 x10⁶ cells/*µ*g of fibre, or from 3 x10⁴ about to about 3 x10⁵ cells/*µ*g of fibre or more particularly from about 4 x10⁴ to about 7 x10⁴ cells/*µ*g of fibre.

In specific embodiments of the invention the typical seeding density is about 40,000 cells/*µ*g of fibre.

A typical cellular aggregate comprises from about 50,000 to about 1x10⁶ cells or from about 100,000 to about 300,000 cells or more particularly from about 150,000 to about 250,000 cells.

The number of cells and/or the amount of fibres can be varied depending on the size and density of the cellular aggregate to be formed. In this way the scaffold can be tailored to the production requirements to give optimal size and fibre/cell density.

Following an appropriate incubation period the cellular aggregate can be directly implanted into a site within a subject. For example the cellular aggregate can be injected at or near a site in need of repair, augmentation or regeneration.

In further embodiments of the disclosure at least two cellular aggregates are loaded into a secondary scaffold. This secondary scaffold can be directly implanted into a site within a subject or alternatively cultured prior to implantation. The incubation of the secondary scaffold prior to implantation allows the primary cellular aggregates to fuse into a larger aggregate, which may be more stable at the time of implantation. These scaffolds are typically incubated for between 12 hours and 7 days. Furthermore the use of a secondary scaffold to contain the primary cellular aggregates provides additional mechanical strength and protection for the primary cellular aggregates during implantation and additionally while the tissue is being regenerated or repaired.

The fibres, primary and secondary scaffolds can be formed of any suitable biocompatible material. A biocompatible material is defined as having the property of being biologically compatible by not producing a toxic, injurious or immunological response in living tissue.

The fibres or scaffolds can be formed of inorganic materials selected from the group consisting of calcium phosphates, calcium carbonates, calcium sulfates or combinations thereof; organic materials selected from the group of biopolymers consisting of a collagen, gelatin, a hyaluronic acid, a proteoglycan, chitin, chitosan, chitosan derivatives, fibrin, dextran, agarose, calcium alginate, silk or combinations thereof, or synthetic polymeric materials selected from the group consisting of aliphatic polyesters, poly(amino acids), poly(propylene fumarate), copoly(ether-esters), polyorthoesters, polyalkylene oxalates, polyamides, polycarbonates, polycaprolactones, poly(iminocarbonates), polyorthoesters, polyoxaesters, polyamidoesters, polyoxaesters containing amine groups, polyanhydrides, polyphospazenes, polyurethanes, hydroxybutyrate, dioxanone, or hydrogels such as polyacrylates, polyvinyl alcohols, polyethylene glycols or polyethylene imines or any co-polymers, blends or chemical derivatives thereof.

The aliphatic polyester can be a polylactic acid or a polyglycolic acid or copolymer or blends thereof.

Suitable scaffolds are formed of copolymers comprising the following monomers or mixtures of polymers and/or copolymers formed thereby: lactic acid, glycolic acid; caprolactone; hydroxybutyrate; dioxanone; orthoesters; orthocarbonates; aminocarbonates.

In preferred embodiments of the invention at least part of the fibres and/or primary scaffold and/or secondary scaffold is a biodegradable, bioresorbable or bioabsorbable material.

In embodiments of the invention in which the fibres and the primary scaffolds are bioresorbable, it is preferable that only a residual amount of scaffold material remains after culture, with the scaffold material having been replaced by the seeded cells and the secreted matrix proteins.

In embodiments of the invention the at least two primary scaffolds are formed of the same material. Alternatively the primary scaffolds are formed of different materials.

In embodiments of the invention the at least two primary scaffolds and the secondary scaffold are formed of the same material. Alternatively at least one of the primary scaffolds is formed of a different material to the secondary scaffold.

In embodiments of the invention the plurality of fibres can consist of fibres made of the same material. Alternatively the plurality of fibres can consist of fibres made of the different materials.

In embodiments of the invention at least part of the fibres and/or primary scaffolds and/or secondary scaffolds are porous. This porosity enables cell migration and nutrient flow throughout the scaffold and prevents the interior of the scaffold becoming anoxic and therefore void of any cellular components.

In particular embodiments of the invention the primary scaffolds are made of non-woven felts.

The methods according to the present invention optionally comprise the step of incubating the cell-seeded fibres and/or primary scaffolds and/or the secondary scaffolds in the presence of a biological agent and/or a chemical agent.

The at least one biological agent and/or a chemical agent can be provided in the culture media. The cells can be exposed to the at least one biological agent and/or a chemical agent constantly or intermittently throughout the duration or the incubation period.

Alternatively the at least one biological and/or chemical agent is associated with at least part of the fibres and/or primary and/or secondary scaffolds.

The at least one biological and/or chemical agent is selected from the group consisting of differentiation agents, growth factors, matrix proteins, peptides, antibodies, enzymes, cytokines, viruses, nucleic acids, peptides, osteogenic factors, chondrogenic factors, immunosuppressants, analgesics or combinations thereof.

For example, the following actives may be used in the culture of cells or additionally delivered with the scaffold implant: members of the transforming factor beta (TGFβ) family, members of the bone morphogenetic protein (BMP) protein family, members of the fibroblast growth factor (FGF) family, platelet derived growth factor, (PDGF), parathyroid hormone related peptide (PTHrP), insulin or insulin-like growth factor, bisphosphonates or pyrophosphates. During the incubation period cells can remain undifferentiated or they can be induced to partially differentiate (ie are "primed") or completely differentiate along at least one appropriate cell lineage. This can be achieved by culturing the cells in media containing differentiation factors.

In embodiments of the invention in which the cells are induced to differentiate along the chondrogenic lineage the cells are cultured in the presence of members of the transforming growth factor beta (TGFβ) family, for example TGFβ-3

According to the disclosure is provided a tissue implant obtainable by the method according to the first or third aspects of the invention.

According to the disclosure is provided a kit of parts comprising at least two primary scaffolds and a second scaffold.

According to the disclosure there is provided a kit of parts comprising at least a plurality of fibres and a second scaffold.

The kit can further comprises a cell source, such as frozen cells.

The kits can further comprise means for isolating cells from an appropriate tissue source, such as surgical instruments for use in tissue biopsies and/or means for comminuting the tissue, for example mechanical devices and/or enzymes.

The kits can further comprise appropriate media and/or tissue culture vessels.

The kits can further comprise at least one biological and/or chemical agent selected from the group consisting of differentiation agents, growth factors, matrix proteins, peptides, antibodies, enzymes, cytokines, viruses, nucleic acids, peptides, osteogenic factors, chondrogenic factors, immunosuppressants, analgesics or combinations thereof.

In embodiments of the invention the extracellular matrix producing cells are stromal cells selected from the group consisting of chondrocytes, osteoblasts, fibroblasts, adipocytes, myoblasts, pericytes, mesenchymal stem cells or any other cell type capable of synthesising an extracellular matrix component, or combinations thereof.

The extracellular matrix component can be a collagen, for example type II collagen.

The extracellular matrix component can be a glycosaminoglycan (GAG), for example chondroitin sulphate, dermatan sulphate, keratin sulphate, heparin sulphate, heparin or hyaluronan. GAG chains may be covalently linked to a protein to form proteoglycans.

The isolated extracellular matrix producing cells may be obtained from donor tissue. The donor tissue may be autologous, allogeneic or xenogeneic and may be derived from any appropriate tissue, including cartilage, bone, skin, tendon, ligament or meniscus. Cells can be obtained from these tissues by standard mechanical methods (e.g biopsy, dissecting, comminuting) and/or enzymatic digestion (e.g collagenase, protease, etc).

In alternative embodiments of the invention, the isolated extracellular matrix producing cells may be derived from a source of primary cells or an established cell line.

In further embodiments of the invention the extracellular matrix producing cells are stem cells or progenitor cells or combinations thereof. The stem cells or progenitor cells can be derived from a fetal or adult source. Suitable sources of these cells include, but are not limited to, bone marrow, blood and umbilical cord blood.

In particular embodiments of the invention the stem cells are mesencyhmal stem cells (MSCs). MSCs are multipotent stem cells that can differentiate into a variety of cell types including osteoblasts, chondrocytes, myocytes, adipocytes and beta-pancreatic islets cells. MSCs have also been shown to transdifferentiate into neuronal cells In further embodiments of the invention the extracellular matrix producing cells can be genetically engineered to constitutively, transiently or inducibly express a gene product beneficial for successful and/or improved transplantation.

The tissues to be augmented, repaired or regenerated by the methods according to the invention can include, but are not limited to, connective tissue such as bone, cartilage, tendon, ligament, meniscus, muscle and adipose. However, other tissues are envisaged.

The subject in which a tissue is being augmented, regenerated or repaired can be a human or non-human animal.

According to an eigth aspect of the invention there are provided methods of forming a tissue implant, a tissue implant therein formed, kits for use in the preparation of the tissue implant and uses of the tissue implant as substantially herein described with reference to the accompanying Figures.

The present invention provides a method of seeding cells on milliscaffolds to produce cellular aggregates without the need for cell-microcarrier aggregate formation.

This present invention further allows the production of pieces of tissue engineered cartilage in a continual process. Cell expansion and differentiation are performed in the same vessel in a continuous process without the requirement of changing culture vessels. This reduces the risk that the sterility of the cultures will be breached, reduces the risk of human error and reduces the number of man hours involved in the culture process. This invention is scalable allowing the large-scale manufacture of tissue engineered cartilage aggregates for use in tissue repair. Unlike inventions within the prior art the invention described herein allows for the treatment of a range of cartilage defect sizes by incorporation into different sized and/or compositions of secondary scaffolds without the need to engineer large pieces of cartilage with the technical difficulties that this encounters. This invention allows the production of a single continuous piece of cartilage by the fusion of smaller pieces of cartilage tissue.

The present invention provides a method of seeding cells on fibrousscaffolds to produce cellular aggregates without the need for cell-microcarrier aggregate formation.

### DESCRIPTION OF THE DRAWINGS

Figure 1: 1 mm x 0.5mm primary scaffolds produced by punching cutting.
Figure 2: A primary scaffold seeded with ovine bone marrow stem cells and cultured for 1 week.
Figure 3: The primary scaffolds illustrated in Figure 2 stained with Safranin O.
Figure 4: A plurality of primary cell-seeded scaffolds loaded into a secondary cup-shaped scaffold.
Figure 5: A primary scaffold seeded with adult human mesenchymal stem cells and cultured for 21 days either (a) with TGF-β3 or (b) without TGF-β3, and then stained with Safranin O.
Figure 6: A primary scaffold seeded with adult ovine chondrocytes, cultured for 21 days, and then stained with Safranin O.

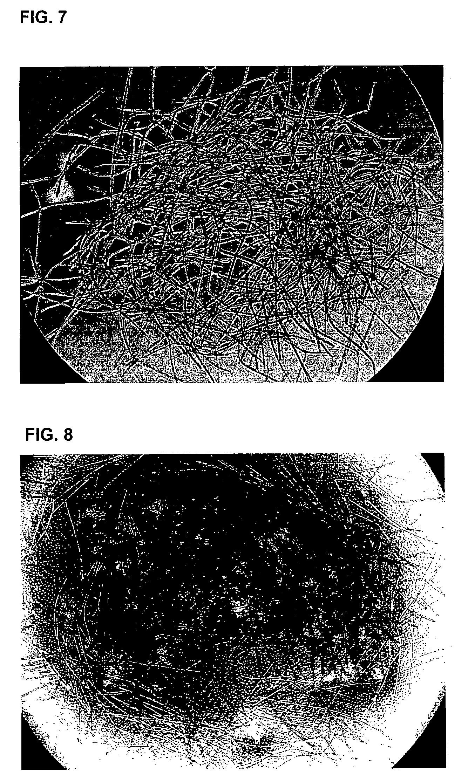

Figure 7: 1-3mm x 0.5mm primary fibres produced by cutting.
Figure 8: A primary fibre-scaffold seeded with human bone marrow stem cells immediately after centrifugation.

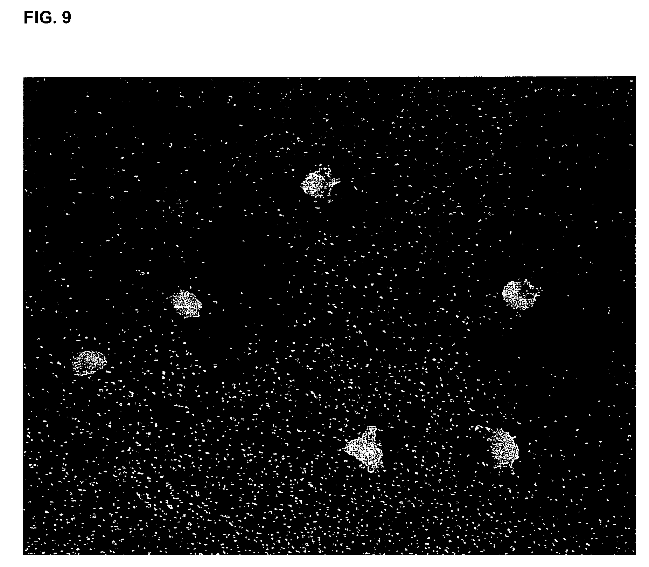

Figure 9: Several fibre-scaffold pellets after 10 days of culture.

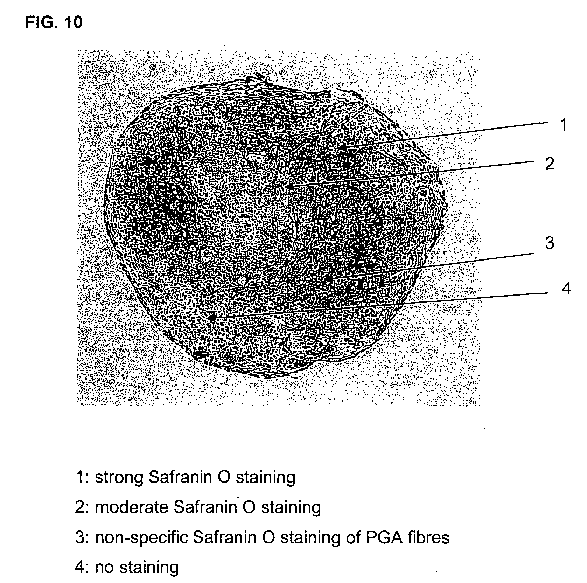

Figure 10: A primary fibre-scaffold seeded with human bone marrow stem cells and cultured for 10 days and stained with Safranin O

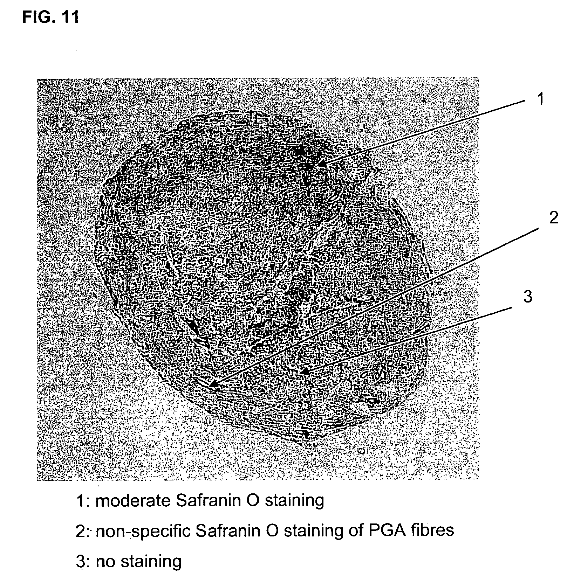

Figure 11: A primary fibre-scaffold seeded with ovine bone marrow stem cells and cultured for 10 days and stained with Safranin O.

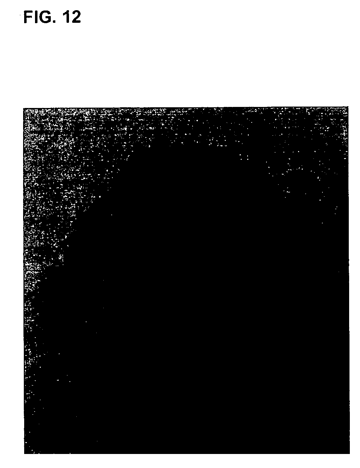

Figure 12: A plurality of primary cell-seeded fibre-scaffolds loaded into a secondary cup-shaped scaffold.

### DETAILED DESCRIPTION OF THE INVENTION

The methods outlined below can be used for the preparation of tissue implants for cartilage repair.

### EXAMPLE 1: Primary scaffolds seeded with ovine bone marrow stem cells

### Step 1: Preparation of primary scaffolds

Polyglycolic acid (PGA) non-woven felt is reinforced with poly(L-lactide-co-glycolic acid (PLLGA) by dipping the felt in a solution of PLLGA and dried. Discs of between about 0.5mm x 1 mm in diameter and between about 0.5 - 3mm in depth were punched out.

The discs were sterilised with a 70:20:10 solution of ethanol:acetone:water, and then incubated in a 50:50 solution of foetal calf serum (FCS) and phosphate buffered saline (PBS) for 2 hours at room temperature to coat the felts with FCS components that aide cell adhesion.

### Step 2: Seeding cells onto primary scaffolds

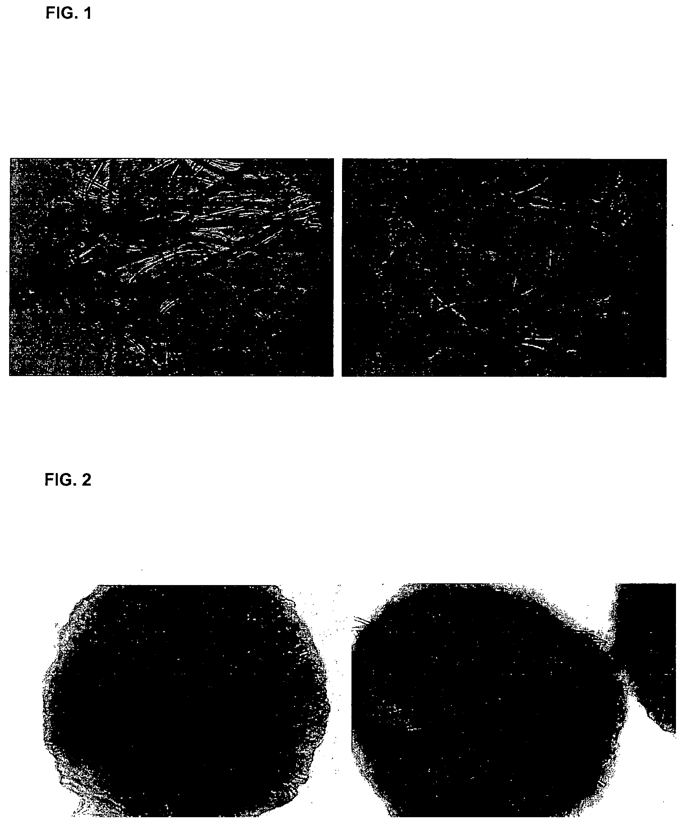

Ovine bone marrow stem cells were seeded onto 45 primary scaffolds at cell number of about 250,000 cells/scaffold. The primary scaffolds were seeded in a falcon tube in a total volume of 5ml α MEM media containing 10% HIFCS, 2mM L-glutamine, 1% non-essential amino acids, 100IU/ml penicillin, 100*µ*g/ml streptomycin, 50*µ*g/ml ascorbic acid and 5ng/ml FGF-2. Cells and scaffolds were cultured overnight on a spiromix platform to constantly bathe the scaffolds in the cell-containing media. After 24 hours the media volume was made up to 25ml and transferred to non-adherent vented tissue culture conical flasks. The cells were cultured with constant agitation on a flat bed shaker for a further week with media being changed every 2-3 days. Figure 2 shows an example of a cell seeded scaffold which has been cultured for 1 week showing the infiltration of cells in the scaffold.

### Step 3: Culture of cell-seeded primary scaffolds in the presence of chondrogenic agents.

After 1 week the cell-seeded scaffolds were transferred to chondrogenic differentiation media consisting of low-glucose DMEM containing 2mM L-glutamine, 1% non-essential amino acids, 100IU/ml penicillin and 100*µ*g/ml streptomycin, containing the following 1x10⁻⁷M dexamethasone, 50*µ*g/ml ascorbic acid, 1×ITS (insulin, transferring, selenious acid), 40*µ*g/ml proline, 1mM sodium pyruvate and 20ng/ml TGFβ3. Scaffolds were cultured for up to 28 days in this media in conical flasks with constant agitation on a flat bed shaker with the media being changed every 3-4 days.

### Step 4: Staining of scaffold using Safranin O

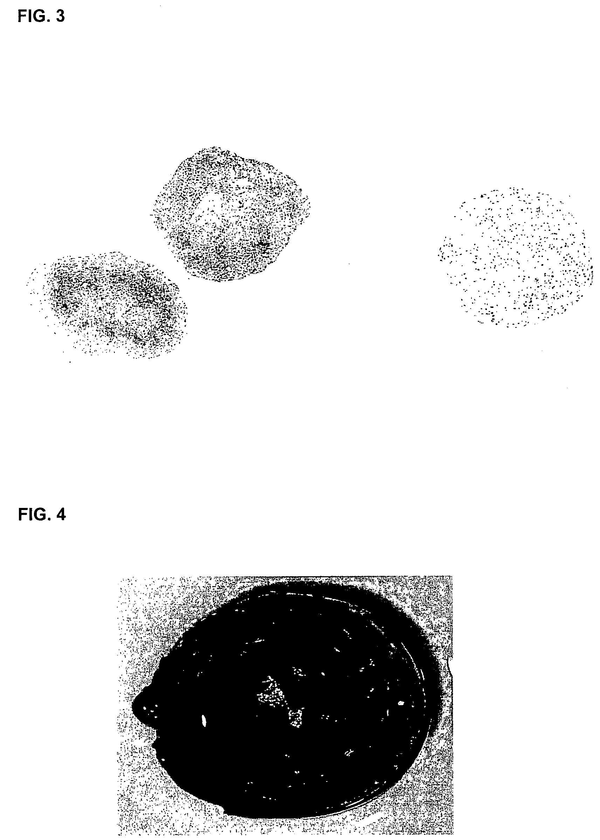

Figure 3 illustrates that the ovine bone marrow stem cells have differentiated during the culture period into chondrocytes which have secreted an extracellular matrix comprises glycosaminoglycans.

### Step 5: Loading of primary scaffolds into secondary scaffolds.

The cell-seeded primary scaffolds were then seeded into a cup-shaped secondary scaffold formed of non-woven PGA felt reinforced with PLLGA and further cultured for up to 7 days prior to implantation/storage. Figure 4 shows an example of a cup scaffold seeded with a 35 of primary cell-seeded scaffolds prior to culture.

### EXAMPLE 2: Primary scaffolds seeded with adult human bone marrow stem cells

### Step 1: Preparation of primary scaffolds

Polyglycolic acid (PGA) non-woven felt is reinforced with poly(L-lactide-co-glycolic acid (PLLGA) by dipping the felt in a solution of PLLGA and dried. Discs of between about 0.5mm x 1 mm in diameter and between about 0.5 - 3mm depth were punched out.

The discs were sterilised with a 70:20:10 solution of ethanol:acetone:water, and then incubated in a 50:50 solution of FCS and PBS for 2 hours at room temperature to coat the felts with FCS components that aide cell adhesion.

### Step 2: Seeding cells onto primary scaffolds

Adult human bone marrow stem cells were resurrected and grown in 2D culture until 90% confluent. The cells were detached from the flask using a trypsin (0.05% w/v) and EDTA (0.02% w/v) solution and then α-MEM media containing 10% FCS was added to the cell suspension to neutralize the activity of trypsin. The cells were counted and the volume adjusted to give a concentration of cells of 250,000 cells per 500 µl of media.

The discs prepared in step 1 were individually placed into single wells in a non-tissue culture treated 2ml deep polypropylene 96-well plate. Aliquots of the media containing 250,000 cells were added to the individual wells. The plates were centrifuged at 400g for 5 minutes to facilitate contact between the cells and the felt.

### Step 3: Culture of cell-seeded primary scaffolds in the presence of a chondrogenic agent.

The cell-seeded discs was incubated in media supplemented with 20 ng/ml TGFß-3 for up to 18 days, with regular media changes.

### Step 4: Staining of scaffold using Safranin O

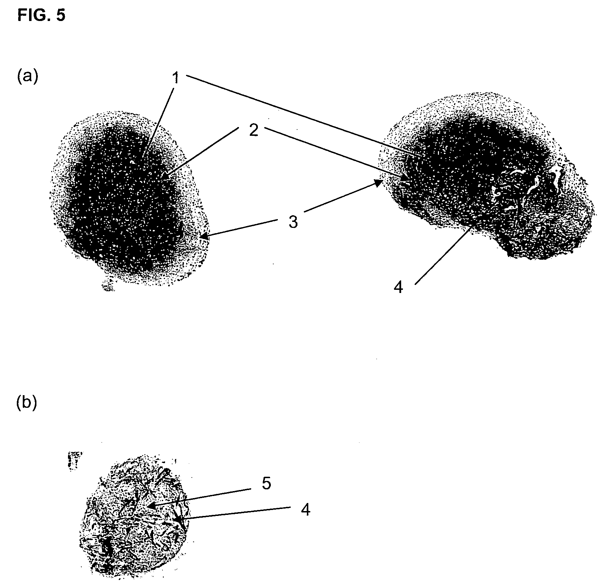

Figure 5 illustrates the staining of the scaffolds with Safranin O after 18 days in culture. The arrows indicate different levels of staining intensity as follows:
1: very strong Safranin O staining
2: strong Safranin O staining
3: moderate Safranin O staining
4: non-specific Safranin O staining of PGA fibres
5: no staining

The high levels of Safranin O staining indicate chondrogenic differentiation and the secretion of a cartilage extracellular matrix. The scaffold has been resorbed by the chondrocytes.

### EXAMPLE 3: Primary scaffolds seeded with adult ovine chondrocytes

### Step 1: Preparation of primary scaffolds

Polyglycolic acid (PGA) non-woven felt is reinforced with poly(L-lactide-co-glycolic acid (PLLGA) by dipping the felt in a solution of PLLGA and dried. Discs of between about 0.5mm x 1 mm in diameter and between about 0.5 - 3mm depth were punched out.

The discs were sterilised with a 70:20:10 solution of ethanol:acetone:water, and then incubated in a 50:50 solution of FCS and PBS for 2 hours at room temperature to coat the felts with FCS components that aide cell adhesion.

### Step 2: Seeding cells onto primary scaffolds

Adult ovine chondrocytes were resurrected and grown in 2D culture until 90% confluent. The cells were detached from the flask using a trypsin (0.05% w/v) and EDTA (0.02% w/v) solution and then α-MEM media containing 10% FCS was added to the cell suspension to neutralize the activity of trypsin. The cells were counted and the volume adjusted to give a concentration of cells of 250,000 cells per 500 µl of media.

The discs prepared in step 1 were individually placed into single wells in a non-tissue culture treated 2ml deep polypropylene 96-well plate. Aliquots of the media containing 250,000 cells were added to the individual wells. The plates were centrifuged at 400g for 5 minutes to facilitate contact between the cells and the felt.

### Step 3: Culture of cell-seeded primary scaffolds in the presence of a chondrogenic agent.

The cell-seeded discs was incubated in media supplemented with 20 ng/ml TGFß-3 for up to 18 days, with regular media changes.

### Step 4: Staining of scaffold using Safranin O

Figure 5 illustrates the staining of the scaffolds with Safranin O after 18 days in culture. The arrows indicate different levels of staining intensity as follows:
1: very strong Safranin O staining
2: strong Safranin O staining
3: moderate Safranin O staining
4: non-specific Safranin O staining of PGA fibres
5: no staining

The high levels of Safranin O staining indicate chondrogenic differentiation and the secretion of a cartilage extracellular matrix. The scaffold has been resorbed by the chondrocytes.

### EXAMPLE 4: The use of a plurality of fibres in the preparation of cell scaffolds

### Step 1: Preparation of primary scaffolds

Polyglycolic acid (PGA) fibres were produced by chopping fibres into 0.5 - 3mm in lengths (FIG. 1).

The fibres were sterilised with a 70:20:10 solution of ethanol:acetone:water, and then incubated in a 50:50 solution of foetal calf serum (FCS) and phosphate buffered saline (PBS) for 2 hours at room temperature to coat the felts with FCS components that aid cell adhesion.

### Step 2: Seeding cells onto primary scaffolds

Sixty five micrograms of fibers prepared according to Step 1 were suspended in a total volume of 5ml DMEM media containing, 2mM L-glutamine, 1% non-essential amino acids, 100IU/ml penicillin, 100*µ*g/ml streptomycin, 50*µ*g/ml ascorbic acid, 1x10⁻⁷M dexamethasone, 50*µ*g/ml ascorbic acid, 1×ITS (insulin, transferrin, selenious acid), 40*µ*g/ml proline, 1mM sodium pyruvate and 20ng/ml TGFβ3. Cells (human and ovine bone marrow derived mesenchymal stem cells) at a concentration of 40,000 cells/*µ*g of fibre (a total cell number of 5x 10⁶ in the 5 ml of fibres and media mentioned above) were added to the fibre suspension. The above suspension was then aliquoted at a volume of 500 *µ*l per sample into individual wells of a sterile 2 ml deep polypropylene 96 well plate and centrifuged at 200g for 5 min to compact the cells and the fibres together (see FIG. 2). This produced 10 cell/fibre pellets. The empty wells surrounding the samples were filled with PBS to increase the humidity in the microenvironment surrounding the samples. The samples were then incubated at 37 °C, 5% CO₂ and 90% humidity for 10 days and resulted in the pellets shown in FIG. 3.

### Step 3: Staining of cell/fibre aggregates using Safranin O

FIG. 4 illustrates that the human bone marrow derived stem cells differentiated during the culture period into chondrocytes which have secreted an extracellular matrix comprises glycosaminoglycans. Figure 5 shows similar results obtained using ovine bone marrow stem cells.

### Step 5: Loading of primary fibre-scaffolds into secondary scaffolds.

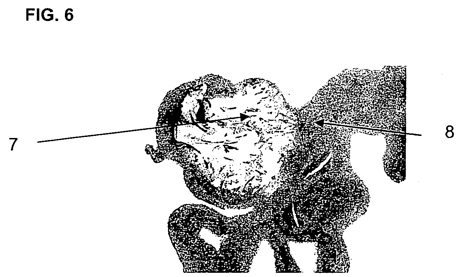

The cell/fibre aggregates were seeded into a cup-shaped secondary scaffold formed of non-woven PGA felt reinforced with PLLGA and further cultured for up to 7 days prior to implantation/storage. Figure 6 shows an example of a cup scaffold seeded with 6 of primary cell-seeded scaffolds prior to culture.

## Claims

1. A method of preparing a tissue implant *in vitro,* said method comprising the steps of;
(a) providing cells isolated from a suitable tissue source;
(b) seeding at least two primary scaffolds with the cells and culturing these scaffolds for a period of time sufficient for the cells to secrete an extracellular matrix; and;
(c) loading the at least two scaffolds obtained in (b) into a secondary scaffold.

2. A method according to claim 1, wherein the extracellular matrix producing cells are stromal cells selected from the group consisting of chondrocytes, osteoblasts, fibroblasts, adipocytes, myoblasts, pericytes, mesenchymal stem cell or any other cell type capable of synthesising an extracellular matrix component or combinations thereof

3. A method according to claim 2, wherein the extracellular matrix component is a collagen or a glycosaminoglycan.

4. A method according to any preceding claim, wherein the cells are stem cells or progenitor cells or combinations thereof.

5. A method according to claim 4, wherein the stem cells are mesenchymal stem cells.

6. A method according to claim 4 or 5, wherein the stem cells or progenitor cells are derived from a fetal or adult source.

7. A method according to any preceding claim, wherein the cells are autogeneic, allogeneic or xenogeneic.

8. A method according to any preceding claim, wherein the primary and secondary scaffolds are formed of inorganic materials selected from the group consisting of calcium phosphates, calcium carbonates, calcium sulfates or combinations thereof; organic materials selected from the group of biopolymers consisting of a collagen, gelatin, a hyaluronic acid, a proteoglycan, chitin, chitosan, chitosan derivatives, fibrin, dextran, agarose, calcium alginate, silk or combinations thereof, or synthetic polymeric materials selected from the group consisting of aliphatic polyesters, poly(amino acids), poly(propylene fumarate), copoly(ether-esters), polyorthoesters, polyalkylene oxalates, polyamides, polycarbonates, polycaprolactones, poly(iminocarbonates), polyoxaesters, polyamidoesters, polyoxaesters containing amine groups, polyanhydrides, polyphosphazenes, polyurethanes, hydroxybutyrate, dioxanone, or hydrogels such as polyacrylates, polyvinyl alcohols, polyethylene glycols or polyethylene imines or any co-polymers, blends or chemical derivatives thereof.

9. A method according to claim 8, wherein the aliphatic polyester is a polylactic acid or a polyglycolic acid or copolymer or blends thereof.

10. A method according to any preceding claim, wherein at least part of the primary and/or secondary scaffold is a biodegradable material.

11. A method according to any preceding claim, wherein the at least part of the primary and/or secondary scaffolds are porous.

12. A method according to any preceding claim, wherein the method further comprises incubating the primary scaffolds and/or the secondary scaffolds in the presence of a biological agent and/or a chemical agent.

13. A method according to any preceding claim, wherein at least one biological and/or chemical agent is associated with at least part of the primary and/or secondary scaffold.

14. A method according to claim 12 or 13, wherein the at least one biological and/or chemical agent is selected from the group consisting of differentiation agents, growth factors, matrix proteins, peptides, antibodies, enzymes, cytokines, viruses, nucleic acids, osteogenic factors, chondrogenic factors, immunosuppressants, analgesics or combinations thereof.

15. A tissue implant obtainable by the method according to any of claims 1 to 14.

## Patentansprüche

1. Ein Verfahren zur In-vitro-Herstellung eines Gewebeimplantats, wobei das Verfahren die folgenden Schritte beinhaltet:
(a) Bereitstellen von aus einer geeigneten Gewebequelle isolierten Zellen;
(b) Beimpfen von mindestens zwei Primärgerüsten mit den Zellen und Kultivieren dieser Gerüste für einen Zeitraum, der ausreicht, dass die Zellen eine extrazelluläre Matrix sezernieren; und
(c) Laden der mindestens zwei in (b) erhaltenen Gerüste in ein Sekundärgerüst.

2. Verfahren gemäß Anspruch 1, wobei die extrazelluläre-Matrix-produzierenden Zellen Bindegewebszellen sind, die aus der Gruppe ausgewählt sind, die aus Folgendem besteht: Chondrozyten, Osteoblasten, Fibroblasten, Adipozyten, Myoblasten, Perizyten, mesenchymalen Stammzellen oder beliebigen anderen Zelltypen, die zum Synthetisieren einer extrazellulären Matrixkomponente fähig sind, oder Kombinationen davon.

3. Verfahren gemäß Anspruch 2, wobei die extrazelluläre Matrixkomponente ein Kollagen oder ein Glykosaminoglykan ist.

4. Verfahren gemäß einem der vorhergehenden Ansprüche, wobei die Zellen Stammzellen oder Vorläuferzellen oder Kombinationen davon sind.

5. Verfahren gemäß Anspruch 4, wobei die Stammzellen mesenchymale Stammzellen sind.

6. Verfahren gemäß Anspruch 4 oder 5, wobei die Stammzellen oder Vorläuferzellen aus einer fetalen oder adulten Quelle abstammen.

7. Verfahren gemäß einem der vorhergehenden Ansprüche, wobei die Zellen autogen, allogen oder xenogen sind.

8. Verfahren gemäß einem der vorhergehenden Ansprüche, wobei die Primär- und Sekundärgerüste gebildet sind aus: anorganischen Materialien, die aus der Gruppe ausgewählt sind, die aus Folgendem besteht: Calciumphosphaten, Calciumcarbonaten, Calciumsulfaten oder Kombinationen davon; organischen Materialien, die aus der Gruppe von Biopolymeren ausgewählt sind, die aus Folgendem besteht: einem Kollagen, Gelatine, einer Hyaluronsäure, einem Proteoglykan, Chitin, Chitosan, Chitosanderivaten, Fibrin, Dextran, Agarose, Calciumalginat, Seide oder Kombinationen davon; oder synthetischen Polymermaterialien, die aus der Gruppe ausgewählt sind, die aus Folgendem besteht: aliphatischen Polyestern, Poly(aminosäuren), Poly(propylenfumarat), Copoly(ether-estern), Polyorthoestern, Polyalkylenoxalaten, Polyamiden, Polycarbonaten, Polycaprolactonen, Poly(iminocarbonaten), Polyoxaestern, Polyamidoestern, Amingruppen-enthaltenden Polyoxaestern, Polyanhydriden, Polyphosphazenen, Polyurethanen, Hydroxybutyrat, Dioxanon oder Hydrogelen wie beispielsweise Polyacrylaten, Polyvinylalkoholen, Polyethylenglykolen oder Polyethyleniminen oder beliebigen Copolymeren, Mischungen oder chemischen Derivaten davon.

9. Verfahren gemäß Anspruch 8, wobei das aliphatische Polyester eine Polymilchsäure oder eine Polyglykolsäure oder ein Copolymer oder Mischungen davon ist.

10. Verfahren gemäß einem der vorhergehenden Ansprüche, wobei mindestens ein Teil des Primär- und/oder des Sekundärgerüsts ein biologisch abbaubares Material ist.

11. Verfahren gemäß einem der vorhergehenden Ansprüche, wobei der mindestens eine Teil der Primär- und/oder der Sekundärgerüste porös ist.

12. Verfahren gemäß einem der vorhergehenden Ansprüche, wobei das Verfahren ferner das Inkubieren der Primärgerüste und/oder der Sekundärgerüste in Gegenwart eines biologischen Wirkstoffs und/oder eines chemischen Wirkstoffs beinhaltet.

13. Verfahren gemäß einem der vorhergehenden Ansprüche, wobei mindestens ein biologischer und/oder chemischer Wirkstoff mit mindestens einem Teil des Primär- und/oder des Sekundärgerüsts assoziiert ist.

14. Verfahren gemäß Anspruch 12 oder 13, wobei der mindestens eine biologische und/oder chemische Wirkstoff aus der Gruppe ausgewählt ist, die aus Folgendem besteht: Differenzierungswirkstoffen, Wachstumsfaktoren, Matrixproteinen, Peptiden, Antikörpern, Enzymen, Zytokinen, Viren, Nukleinsäuren, osteogenen Faktoren, chondrogenen Faktoren, Immunsuppressiva, Analgetika oder Kombinationen davon.

15. Ein Gewebeimplantat, das durch das Verfahren gemäß einem der Ansprüche 1 bis 14 erhalten werden kann.

## Revendications

1. Un procédé de préparation d'un implant tissulaire in vitro, ledit procédé comprenant les étapes consistant à ;
(a) fournir des cellules isolées à partir d'une source tissulaire appropriée ;
(b) ensemencer au moins deux échafaudages primaires avec les cellules et cultiver ces échafaudages pendant une période de temps suffisante pour que les cellules sécrètent une matrice extracellulaire ; et ;
(c) charger les au moins deux échafaudages obtenus en b) dans un échafaudage secondaire.

2. Un procédé selon la revendication 1, dans lequel les cellules produisant la matrice extracellulaire sont des cellules stromales sélectionnées dans le groupe constitué de chondrocytes, d'ostéoblastes, de fibroblastes, d'adipocytes, de myoblastes, de péricytes, de cellule souche mésenchymateuse ou tout autre type de cellule capable de synthétiser un composant de matrice extracellulaire ou des combinaisons de ceux-ci.

3. Un procédé selon la revendication 2, dans lequel le composant de matrice extracellulaire est un collagène ou un glycosaminoglycane.

4. Un procédé selon n'importe quelle revendication précédente, dans lequel les cellules sont des cellules souches ou des cellules progénitrices ou des combinaisons de celles-ci.

5. Un procédé selon la revendication 4, dans lequel les cellules souches sont des cellules souches mésenchymateuses.

6. Un procédé selon la revendication 4 ou la revendication 5, dans lequel les cellules souches ou les cellules progénitrices sont dérivées d'une source foetale ou adulte.

7. Un procédé selon n'importe quelle revendication précédente, dans lequel les cellules sont autologues, allogéniques ou xénogéniques.

8. Un procédé selon n'importe quelle revendication précédente, dans lequel les échafaudages primaires et secondaires sont formées de matériaux inorganiques sélectionnés dans le groupe constitué de phosphates de calcium, de carbonates de calcium, de sulfates de calcium ou de combinaisons de ceux-ci ; de matériaux organiques sélectionnés dans le groupe de biopolymères constitué d'un collagène, de gélatine, d'un acide hyaluronique, d'un protéoglycane, de chitine, de chitosane, de dérivés du chitosane, de fibrine, de dextrane, d'agarose, d'alginate de calcium, de soie ou de combinaisons de ceux-ci, ou de matériaux polymères synthétiques sélectionnés dans le groupe constitué de polyesters aliphatiques, de poly(acides aminés), de poly(propylène fumarate), de copoly(éther-esters), de polyorthoesters, d'oxalates de polyalkylène, de polyamides, de polycarbonates, de polycaprolactones, de poly(iminocarbonates), de polyoxaesters, de polyamidoesters, de polyoxaesters contenant des groupes amine, de polyanhydrides, de polyphosphazènes, de polyuréthanes, d'hydroxybutyrate, de dioxanone, ou d'hydrogels tels que des polyacrylates, des alcools polyvinyliques, des polyéthylènes glycols ou des polyéthylènes imines ou tous copolymères, mélanges ou dérivés chimiques de ceux-ci.

9. Un procédé selon la revendication 8, dans lequel le polyester aliphatique est un acide polylactique ou un acide polyglycolique ou un copolymère ou des mélanges de ceux-ci.

10. Un procédé selon n'importe quelle revendication précédente, dans lequel au moins une partie de l'échafaudage primaire et/ou secondaire est un matériau biodégradable.

11. Un procédé selon n'importe quelle revendication précédente, dans lequel l'au moins une partie des échafaudages primaires et/ou secondaires sont poreux.

12. Un procédé selon n'importe quelle revendication précédente, dans lequel le procédé comprend en outre l'incubation des échafaudages primaires et/ou les échafaudages secondaires en présence d'un agent biologique et/ou d'un agent chimique.

13. Un procédé selon n'importe quelle revendication précédente, dans lequel au moins un agent biologique et/ou chimique est associé à au moins une partie de l'échafaudage primaire et/ou secondaire.

14. Un procédé selon la revendication 12 ou la revendication 13, dans lequel l'au moins un agent biologique et/ou chimique est sélectionné dans le groupe constitué d'agents de différentiation, de facteurs de croissance, de protéines de matrice, de peptides, d'anticorps, d'enzymes, de cytokines, de virus, d'acides nucléiques, de facteurs ostéogènes, de facteurs chondrogéniques, d'immunosuppresseurs, d'analgésiques ou de combinaisons de ceux-ci.

15. Un implant tissulaire pouvant être obtenu par le procédé selon n'importe lesquelles des revendications 1 à 14.
